# EUROPEAN PATENT SPECIFICATION

(11) **EP 2 724 149 B1**
(45) Date of publication and mention of the grant of the patent: **14.10.2015**
(21) Application number: 12741048.8
(22) Date of filing: 21.06.2012
(51) Int. Cl.: G01N 27/12, G01N 27/22, G01N 33/24

(54) **MATRIC POTENTIAL SENSOR AND RELATED METHODS**
MATRIXPOTENZIALSENSOR UND ZUGEHÖRIGE VERFAHREN
CAPTEUR DE POTENTIEL CAPILLAIRE ET PROCÉDÉS CORRESPONDANTS

(30) Priority: 22.06.2011 GB 201110550
(43) Date of publication of application: 30.04.2014
(73) Proprietor: Delta-T Devices Limited, Burwell Cambridgeshire CB25 0EJ (GB)
(72) Inventor: GOODCHILD, Martin Scott, Burwell Cambridgeshire CB25 0EJ (GB); JENKINS, Malcolm David, Burwell Cambridgeshire CB25 0EJ (GB); BUREK, Kazimierz Jan, Burwell Cambridgeshire CB25 0EJ (GB); JANES, Stuart Andrew, Burwell Cambridgeshire CB25 0EJ (GB)
(74) Representative: Gosnall, Toby
(86) International application number: PCT/GB2012/051446
(87) International publication number: WO 2012/175976

(56) References cited:
- US-A- 4 137 931
- US-A- 4 909 070
- US-A1- 2006 290 360
- WHALLEY W R ET AL: "Measurement of low matric potentials with porous matrix sensors and water-filled tensiometers", SOIL SCIENCE SOCIETY OF AMERICA JOURNAL NOVEMBER-DECEMBER 2009 SOIL SCIENCE SOCIETY OF AMERICA USA, vol. 73, no. 6, November 2009 (2009-11), pages 1796-1803, XP002685216, DOI: DOI:10.2136/SSSAJ2008.0400

## Description

### Field of the invention

This invention relates to a sensor and the related field of improving the accuracy and/or reliability of a sensor. In particular, but not exclusively, the sensor is a medium moisture sensor. Further, and again not exclusively, the invention may relate to a matric potential sensor arranged to measure a mediums ability to take up moisture. Specifically, and again not exclusively, the invention may relate to a sensor containing ceramic portions arranged to take up water.

### Background of the invention

Whilst the field of soil-moisture content measurement has been developed for several decades, the field of matric potential measurement is more recent and tries to measure the ability of a medium, typically soil, to take up and retain water. The matric potential of a medium gives a measurement of the mediums's attraction for water which may be thought of as the agility of that medium to retain water. As such, knowing the matric potential of a medium gives a useful measurement in determining the availability of water from that medium.

It is convenient to describe the background of this idea in relation to the measurement of soil water content but the invention has wider applicability. For example the sensor may be used in any of the non-exhaustive list of substrates and/or mediums: cotton wool, mineral wool, sand, rock-wool, volcanic material, plant growing media, concrete, building material, pharmaceutical materials and the like. Further, the moisture content sensor may also find application for measuring in the, non-exhaustive, list of applications: environmental monitoring, irrigation monitoring, irrigation control, crop yield optimisation, flood control, damp measurement, building subsidence, refuse compost monitoring and drug manufacturing in the pharmaceutical industry.

As such, a medium such as clay which has relatively small pores has an ability to retain water which is higher than that of a coarse medium, such as sandy soils, with relatively large pores. Knowing the matric potential of a medium at a particular water content can be used to determine, for example when to irrigate, etc. and help to avoid over irrigation thereby conserving water. Furthermore, measuring matric potential can provide a soil-type independent assessment of the soil moisture that can be reliably used to avoid water stress conditions that could be detrimental to plant growth and result in reduced crop yields.

Such matric potential measurement systems originally used water-filled tensiometers which suffered from a number of problems, including: complex to operate; limited range of measurement; attract the roots of nearby plants due to constant water source; cannot operate in below freezing conditions; need for degassed water.

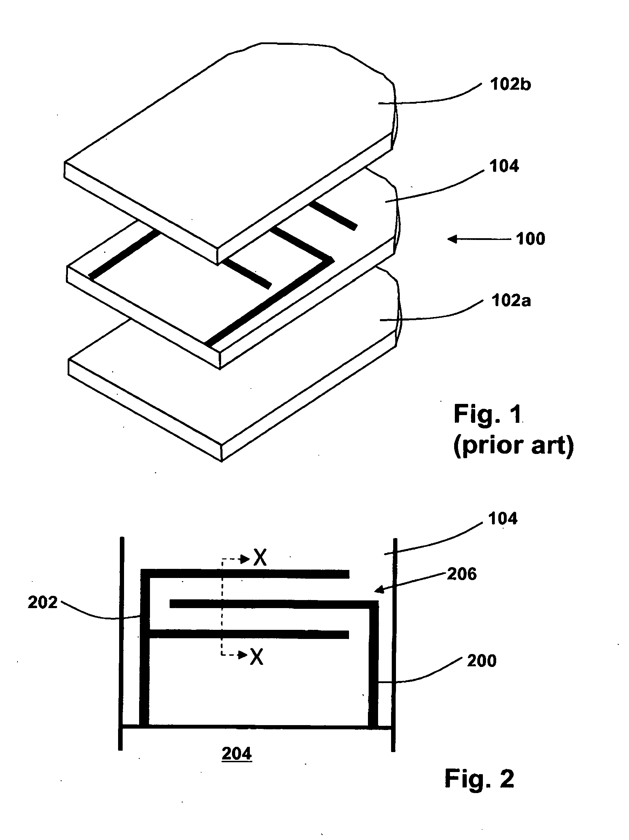

More recently, matric potential sensors comprising a portion of ceramic 102a, 102b (as shown in Figure 1 of the accompanying drawings) have been used wherein the ceramic is arranged to draw water from and equilibrate with the surrounding medium. An example of such a sensor is shown in US 7 042 234 which also provides a detailed summary of the shortcomings of a water-filled tensiometer.

Water has a high dielectric constant (εᵣ = 81) and as such it is known that it should be prevented from entering gaps within the sensor. Prior art sensors have tried to ensure that the ceramic portions, as exemplified by portions 102a, 102b, are as flat as possible in order to give a flat surface to which a sensor can be mounted.

US 4,137,931 discloses a matric potential sensor comprising (a) a housing including a porous wall adapted to be located underground, (b) spaced electrodes carried in the housing to be coupled to a source of electrical current, and (c) granular material located to pass electrical current within the housing and between the electrodes so that the current flow will vary as a function of the moisture content of the granular material, said content adapted to vary as a function of the matric potential of the soil surrounding the housing in response to moisture transmission through the porous housing into the surrounding soil.

### Summary of the invention

According to a first aspect of the invention there is provided a matric potential sensor arranged to measure the matric potential of a medium. Typically the sensor comprises a porous material portion, arranged to absorb moisture from the medium into which, in use, it is inserted. Further, the sensor will typically comprise an electrode element and generally wherein a compliant material is provided between the porous material portion and the electrode element. Embodiments will typically use a non-porous material as the compliant material.

Typically, in the prior art, there has been a drive to place the electrode element as close as possible to any porous material portion in order that any available signal is not attenuated more than might be desired. The skilled person will appreciate that some sensors are typically used to measure a capacitance change which may be on the order of pF. In view of this small change, there is generally a drive to keep as much signal as possible. However, it has been found that the use of a compliant non-porous material can increase the performance of a sensor by removing voids, which typically occur in regions of high sensitivity (eg beside the electrode element) that could previously have affected the readings given by a sensor despite the attenuation of the signal that may be caused by the presence of the compliant material.

The compliant material may be thought of as being an interstitial filler. As such, embodiments will typically provide a compliant non-porous interstitial filler between the porous material portion and the electrode element.

In particular voids which occur due to manufacturing tolerances cause variations between different sensors from the same production run and thereby reduce the repeatability between sensors. The problem is further exacerbated by the fact that the voids may or may not contain water and as such the effect on the reading from the sensor swings between a maximum and minimum creating a random effect on the reading. It is has been found that filling these voids with a compliant non-porous material mitigates these issues by introducing a fixed dielectric constant which does not vary significantly with water content.

The compliant material may be a hydrophobic material which is advantageous to as it helps to prevent the ingress of water. In such embodiments, the compliant material may or may not be an open-cell foam.

In other alternative embodiments, or additionally, the compliant material may be a closed-cell foam. Such an arrangement also aids prevention of water ingress since it also prevents water from entering the compliant material and may also ensure the compliant material is more compressible since it will contain sealed voids.

However, the compliant material will generally be selected to be compatible with the environment in which the matric potential sensor is arranged to be used.

In some embodiments, the compliant material may be a silicone rubber and further be a food-grade silicone rubber. A suitable material can be supplied from J-Flex Rubber Products, Unit 1, London Road Business Park, Retford, Nottinghamshire, DN22 6HG.

Typically the compliant material may have a thickness of roughly in the range of 50µm to 250µm. In some embodiments the range may be 100µm to 200µm. In one particular embodiment the compliant material has a thickness of substantially 150µm.

In some embodiments, the compliant material has a relative permittivity of substantially in the range 2 to 5. In other embodiments, the relative permittivity may be roughly 3.

The compliant material may be provided as any of the following: one or more sheets; a sleeve.

Typically, the porous material portion is a ceramic material. In one embodiment, the ceramic material (which may be termed a ceramic matrix) is Alpha alumina available from suppliers such as Fairey Industrial Ceramics Ltd, Lower Milehouse Lane, Newcastle-under-Lyme, ST5 9BT. Other materials also available from this supplier include Pyrolith^{™} (alumino-silicate particles bonded by glass) or Celloton^{™} (alumina particles bonded by glass or porcelain mullite).

In some embodiments, the pore sizes within the ceramic are in the range 0.1µm to 500µm. Other embodiments may have a range of between roughly 50µm and 150µm.

The porosity of the ceramic may be in the range of substantially 20% to 50%. In addition to these values, the porosity may for example be roughly 25%, 30%, 35%, 40% or 45%.

More than one portion of porous material may be provided. In one embodiment, two portions of porous material are provided. The material from which the portions of porous material is fabricated may be varied between portions.

In other embodiments, the porous material may not be a ceramic material and may instead be any one of the following: ceramic frits, hydrophilic porous plastics, resins, fibre wicks, or any other open-cell structure.

Should the porous material be of a material other than a ceramic material then the pore size and distribution may be substantially the same as discussed in relation to the ceramic material.

The compliant material and/or porous material may be mirrored about an axis, plane or the like. The compliant material and/or porous materials may, in particular, be mirrored about the electrode element (ie be the same either side of the sensor).

The electrode element may comprise a substantially planar element. In such an embodiment a porous material portion may be provided on each side of the planar electrode element.

In embodiments, which have more than one portion of porous material, one or more dimensions of the porous material may be varied when compared with one another. For example, the pore size of at least first and second portions of porous material may be varied with respect to one another. Such embodiments are believed useful in that such a sensor should allow the range over which the sensor can be operated to be extended.

In some embodiments, the electrode element comprises a circuit board comprising one or more tracks. In circuit boards according to known fabrication techniques, variations in the thickness of tracks tracks of up to 10µm have been recorded. Also, in such circuit boards, the weave of fibres within the fabric of the board can also lead to variations in the surface of the board. As such, an advantage of embodiments that have the compliant material is that such variations can be absorbed thus helping to remove pockets that might otherwise hold water.

Typically, the or each track of the electrode element will be arranged to pass an electric current. At least one track, and typically two tracks, provided on the sensor may be arranged to form a plate of a capacitor. As such, sensing electronics may be arranged to measure a variation in capacitance of the sensor. The capacitance of the sensor may be arranged to be caused to vary by the moisture content of the surrounding soil and/or absorbent portions.

In alternative, or additional, embodiments the electrode element may be provided as one or more pins. Typically, a plurality of pins are provided.

In some embodiments, the porous material may contain recesses in which the electrode elements are provided and the compliant material is conveniently positioned within the recesses.

In some embodiments an adhesive may be used to secure portions of the porous material in place. In particular, the adhesive may be arranged to secure portions of porous material to one another.

Typically, should an adhesive be used, it will be used in a region of the porous material removed (ie distant) from the electrode element.

The compliant material may be compressed between the electrode element and the porous material.

In some embodiments the matric potential sensor may be a di-electric tensiometer.

According to a second aspect of the invention there is provided a method of fabricating a sensor comprising placing a layer of compliant non-porous material between an electrode element and portion of absorbent material.

The skilled person will appreciate that features described in relation to any one of the above aspects of the invention may be applied, mutatis mutandis, to any other of the aspects of the invention.

### Brief description of the drawings

There now follows by way of example only a detailed description of embodiments of the present invention with reference to the accompanying drawings in which:
**Figure 1** **(prior art)** shows an exploded perspective view of a sensor;
**Figure 2** shows a plan view of a circuit board element of an embodiment of the invention and of the prior art;
**Figure 3** **(prior art)** shows a section along line XX of Figure 2 in the sensor of Figure 1 in a dry condition;
**Figure 4** **(prior art)** shows a section along line XX of Figure 2 in the sensor of Figure 1 in a partially wetted condition where the ceramics are saturated;
**Figure 5** **(prior art)** shows a section along line XX of Figure 2 in the sensor of Figure 1 in a saturated condition;
**Figure 6** shows an exploded perspective view of a sensor according to an embodiment of the invention;
**Figure 7** shows a section along line XX of Figure 2 in the sensor of Figure 6 in a dry condition;
**Figure 8** **(prior art)** shows experimental results showing the performance of the theoretical model and the measured performance of a sensor validated by an SWT4 water filled tensiometer according to the embodiment of Figure 1;
**Figure 9** shows the modelling flow-chart highlighting the model used to generate the graphs of Figures 8 and 10;
**Figure 10** shows experimental results showing the performance of the theoretical model and the measured performance of a sensor validated by an SWT4 water filled tensiometer according to the embodiment of Figure 6;
**Figure 11** shows further experimental results;
**Figure 12** **(prior art)** shows a cross section through a further prior art sensor;
**Figure 13** **(prior art)** shows a section through line YY of Figure 12;
**Figure 14** shows a section, similar to that shown in Figure 13, but for an embodiment of the invention;
**Figure 15** shows a further embodiment of the invention which is similar to Figure 14;
**Figure 16** shows a further embodiment of the invention; and
**Figure 17** shows yet a further embodiment of the invention.

### Detailed description of the drawings

The prior art sensor 100 of Figure 1 comprises a porous material portion 102a, 102b (which in this case is ceramic) provided on each side of a sensor element which in this case is a circuit board 104 which can be used to measure the moisture content within the ceramic portions 102a, 102b. In this embodiment, the circuit board 104 provides an electrode element.

The circuit board 104 is of a conventional multi-layer design. In the embodiment being described, the circuit board 104 comprises two tracks 200, 202 which connect to sensing electronics 204. Each of the tracks 200,202 provides the plate of a capacitor 206, the capacitance of which will vary according to the dielectric constant of the surrounding medium (ie the ceramic portions 102a, 102b). The dielectric constant of the surrounding medium is affected by the amount of water held within the ceramic portions 102a, 102b.

The sensing electronics 204 may be mounted off the circuit board 104 as is the case in the embodiment being described in relation to Figure 1, but in other embodiments the sensing electronics may be mounted on the circuit board 104. Indeed, it would be possible for some of the sensing electronics to be mounted upon the circuit board 104 and some of the sensing electronics to be mounted off the circuit board 104.

The sensing electronics 204 can use the capacitance value of the capacitor 206 to determine the moisture content of ceramic portions 102a, 102b and thereby the surrounding medium. The sensing electronics may be as described in EP 1 836 483 which is hereby incorporated by reference.

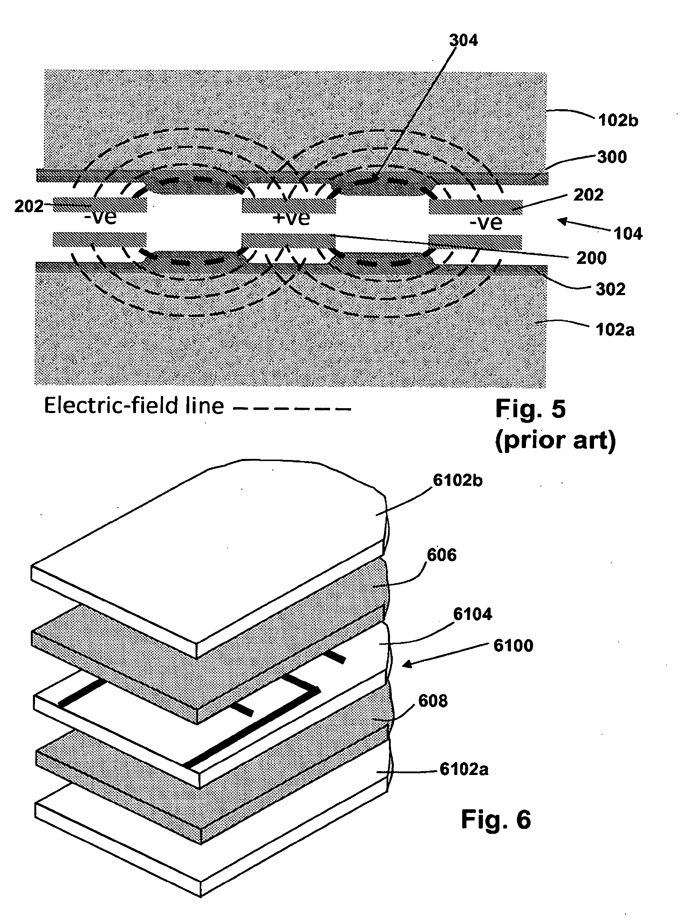

However, to summarise the teachings of EP 1 836 483 a signal source generates a signal, which is passed through a sensing impedance and a Pi-circuit connected in series, into a sensor. The sensor may be that as shown in Figures 1 or 6. The sensor together with the medium into which the sensor is inserted provide a varying complex impedance. The sensing impedance together with the Pi-circuit and the sensor/medium combination form a potential divider across which sensing electronics (such as 204) can make measurements to determine properties of the soil.

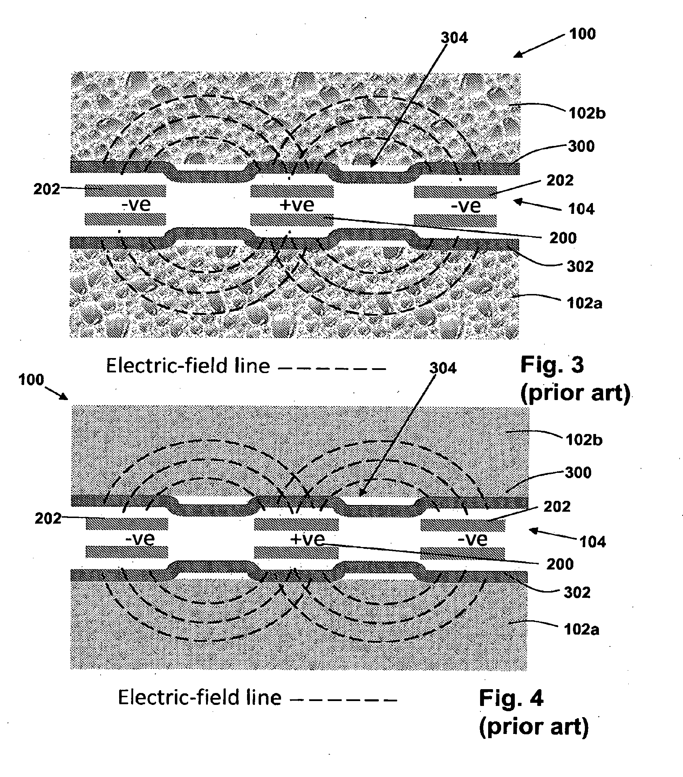

Turning to Figure 3, the circuit board 104 can be seen sandwiched between the two ceramic portions 102a, 120b. In this Figure, the circuit board 104 is shown in more detail and in addition to the tracks 200, 202 the outer surfaces 300, 302 are shown. This outer surface would typically be a coating applied over the surface of the circuit board 104 which might be roughly 200µm of FR4 PCB material. In other embodiments, the track might be exposed or covered with a thinner coating.

Looking at Figure 3, it can be seen that the upper and lower surfaces (as seen in the Figure) of the circuit board 104 are not planar, although at a macro-scale the surfaces of the circuit board 104 approximate to planar. As such, depressions eg 304 occur in the surface of the circuit board 104, perhaps in regions between the tracks 200, 202. In general, any surface imperfection in circuit board 104 may provide a depression or bump (for example caused by the weave of fibres within the board) which could alter the dimensional relationship between the ceramic portions 102a, 102b and the circuit board 104.

Turning to Figure 4, the sensor 100 of Figure 3 is shown but with the portions of ceramic 102a, 102b saturated with water. However, it will be seen that the depressions 304 remain unfilled with water despite the fact that the ceramic portions 102a, 102b are saturated.

Thus, the depressions 304 as seen in Figures 3 and 4 provide further volumes that can hold water and as such, the sensor can absorb further water once the ceramic portions 102a, 102b have become saturated and this situation is represented in Figure 5. This extra water, whilst potentially of small volume, can fill a region, which due to its proximity to the circuit board 104, is of very high sensitivity and means that the sensor does not behave as it theoretically should.

Figure 6 shows a sensor 6100 according to an embodiment of the present invention in which like parts when compared with Figure 1 are shown with like reference numerals but with a 6 placed in front of the earlier reference number. In addition to the layers shown in Figure 1, the sensor 6100 of Figure 6, comprises a layer of compliant material 606, 608 provided on each side of the circuit board 6104. Thus, a layer of compliant material, typically non-porous 606, 608 is sandwiched between the portion of porous material 6102a, 6102b and the circuit board 6104. The layer of porous material 6102a may be thought of as a first porous layer and the layer of porous material 6102b may be thought of as a second porous layer.

The thicknesses of the layers are not shown to scale and, in particular, the layers of compliant material 606, 608 are shown thicker than is actually likely to be the case; it will be appreciated the thicker this layer is made the lower the change of capacitance that will be measured by the sensing electronics 6204.

In this embodiment, the compliant material layers are each roughly 150µm thick portions of food-grade silicone rubber. In other embodiments, the compliant material may be any other suitable closed cell foam, hydrophobic material, or the like.

Typically the sensor is secured together by a mechanical fixing means. For example, one or more bolts, screws or other rods could be passed through the sensor and used to clamp the components together. In other embodiments, a cage could be created to hold the components together. In the embodiment being described a plastic friction rivet is used. Some embodiments of the invention may include a filler, sealant, or the like being added to any holes created through the sensor to accommodate fixing means in order that gaps are not created which could hold further water.

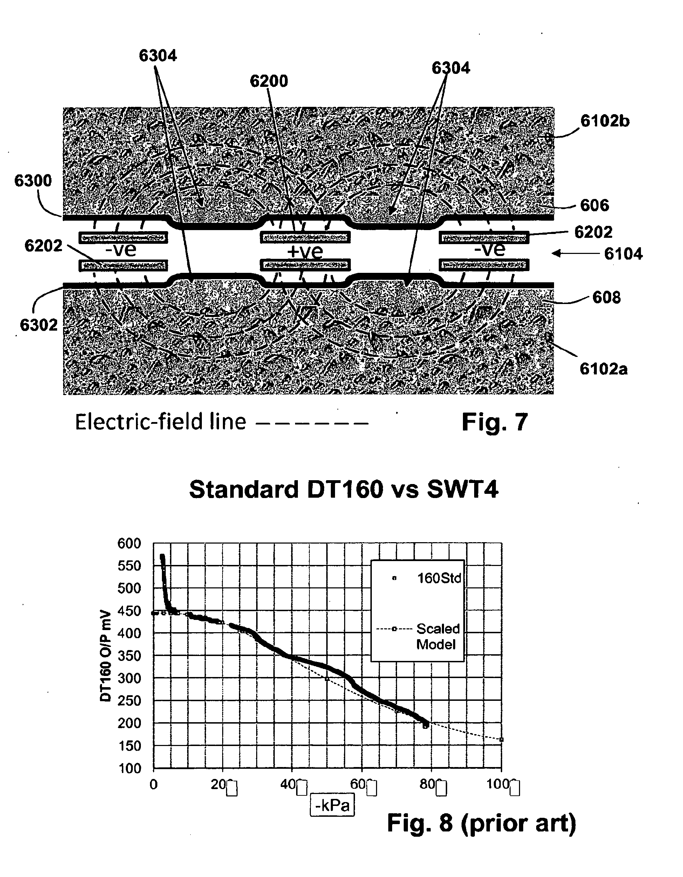

As can be seen from Figure 7, the compliant material 606, 608 is compressed between the portions of ceramic 6102a, 6102b and the sensor circuit board 6104 such that the depressions 6304 become filled with the compliant material 606, 608. As such, embodiments of the invention that have a layer of compliant material tend to have more uniform properties since there are no pockets (formed by the depressions) that can hold water. Water held in such pockets may well be unabsorbed / absorbed at different pressures than water held in the portions of porous material 102a, 102b which affects the performance of the sensor 104.

Figure 8 shows a comparison of the performance of a theoretical model shown in dashed line and the measured performance of the sensor, (validated by an SWT4 water filled tensiometer) according to the prior art and as described in relation to Figure 1. It will be seen that there is a significant deviation of the measured performance at high saturations; that is with relatively high pressure (ie close to zero since the measured pressures are always negative when referring to matric potential measurements). It is believed that this is due to the departure from the model caused due to pockets 304 filling with water as described in relation to Figures 3 to 5, furthermore, water unabsorbed / absorbed at a different pressures may explain the divergence of the graph of Figure 8 at around -50kPa.

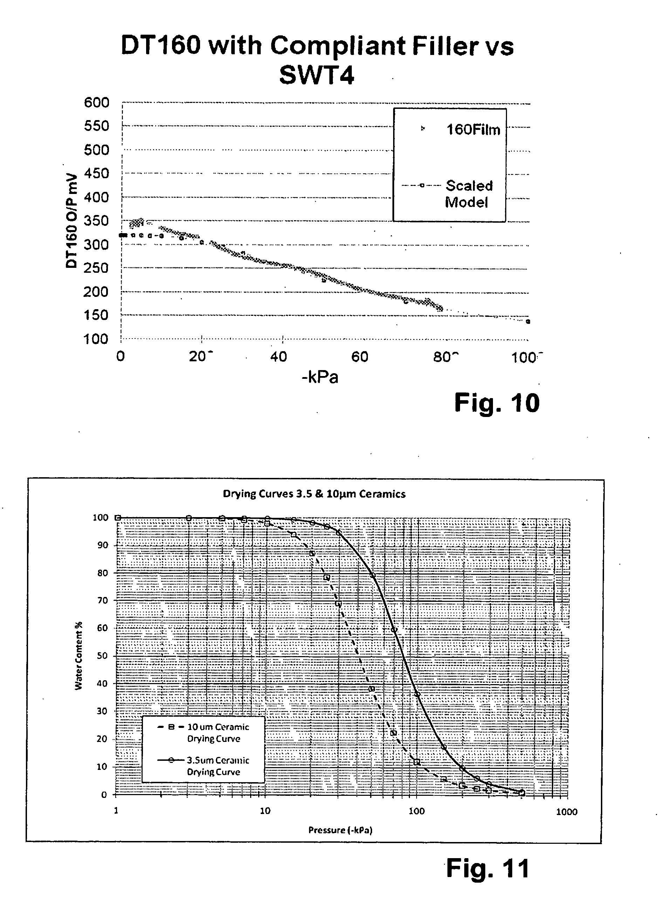

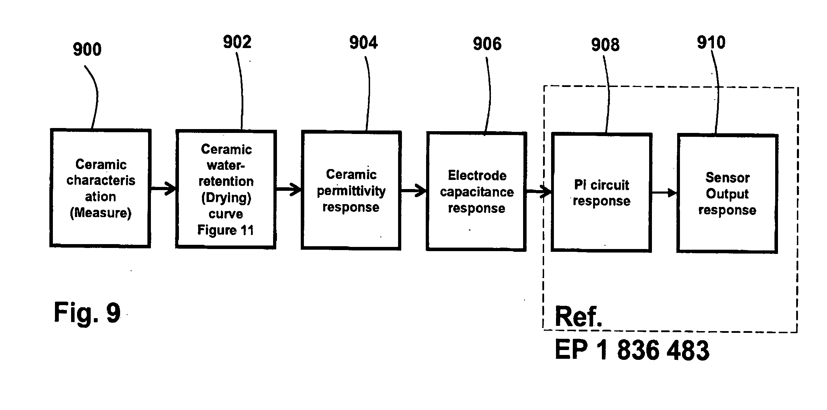

In creating the model, the process outlined in relation to Figure 9 is used in which the ceramic characteristics 900 (ie the characteristics of the porous material) are used to create ceramic water retention drying curves 902 (examples shown in Figure 11). Such drying curves can be determined through routine experimental analysis to determine the water content of the ceramics as a function of pressure.

In turn, the water content of the ceramic (ie the porous material portions) can be used to determine the permittivity as a function of pressure - step 904. In turn the permittivity of the electrode at a given pressure can be used to determine the expected capacitance response (ie from the capacitor 206 of the electrode element) for a given pressure 906.

In this regard, and as an example of one embodiment, it is noted that the relative permittivity of water is 81 (εᵣ = 81). A dry ceramic may have a permittivity of roughly 5 or 6 ((εᵣ = 5 or 6) and a saturated ceramic might have a permittivity of roughly 25 (εᵣ = 25). As such, the skilled person will appreciate that water has markedly greater effect than even a saturated ceramic. Other porous materials are likely to have similar relative permittivity's to that of a ceramic material.

Further, for a given capacitance from the capacitor 206 of the electrode element it is possible to determine the expected Pi-circuit response 908 and further to determine the output from the system embodying both the matric potential sensor 6100 and the associated sensing electronics 204; ie to determine a response as a function of pressure and therefore matric potential once the ceramic has equilibrated with the surrounding medium.

The skilled person will appreciate that the typical change in capacitance that is likely to be shown in the capacitance of the capacitor 206 will be on the order of a few pF. As such, the changes in permittivity between water and a saturated porous material can have a large effect.

In the embodiment being described the Pi-circuit response 908 and the sensor output response 910 may be as shown in Figure 11 of EP 1 836 483 to which the reader is referred for further information.

Figure 10 shows a comparison of the performance of the same theoretical model vs. the measured performance of a sensor, (validated by an SWT4 water filled tensiometer) according to the embodiment of Figures 6 to 7. The skilled person will also note that y-axis intersection occurs at around 300-350mV as opposed to the approximately 450mV of Figure 8. This difference in y-axis intersection exemplifies the signal loss that is caused by the use of the compliant material. To generate Figure 10 a compliant material (ie compliant filler) of around 300µm was used. As the thickness of the compliant material is reduced the y-axis intersection moves closer to the intersection with the y-axis shown in Figure 8.

It is noted that the measured model follows the performance of the theoretical model far more closely at high saturations and there is also less of a deviation at around - 50 kPa.

In the embodiment of Figures 6 to 7 the portion of ceramic 6102a has a different pore size to the portion of ceramic 6102b. In particular, the pore size of the portion of ceramic 6102a is roughly 3.5µm whereas the portion of ceramic 6120b is roughly 10µm. Varying the pore sizes has the effect of altering the tension at which water is taken and given up by the portion of ceramic which has the effect of extending the range of which data is provided by the sensor. In particular, Figure 11 shows the drying curves for portions of ceramic having these pore sizes in which the lower curve represents 10µm and the upper curve represents 3.5µm. It is noted that, due to the larger pore size, the water content of 10µm sized ceramic changes significantly at higher pressures than that having a 3.5µm pore size.

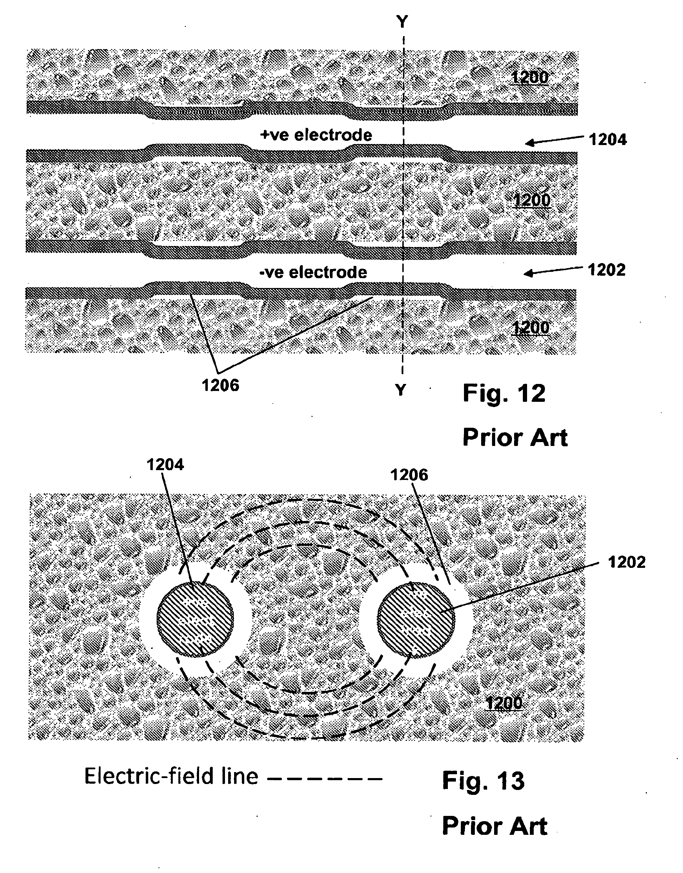

Figure 12 shows a further example of a prior art sensor, in a dry condition, which comprises a porous material portion 1200 in which two metallic pins 1202, 1204 have been inserted. It can be seen that the pins 1202, 1204 are contoured such that there are gaps 1206 between the pins 1200, 1202 and the porous material portions 1200 which provide what may be thought of as an air filled interface. Figure 13 shows similar components to Figure 12 but along the sectional line YY of Figure 12.

The readings given by the sensor of Figures 12 and 13 is affected by the gaps 1206 in the same way that the presence / absence of water within the gaps 1206 can significantly effect the reading given by the sensor as described in relation to Figures 1 to 11

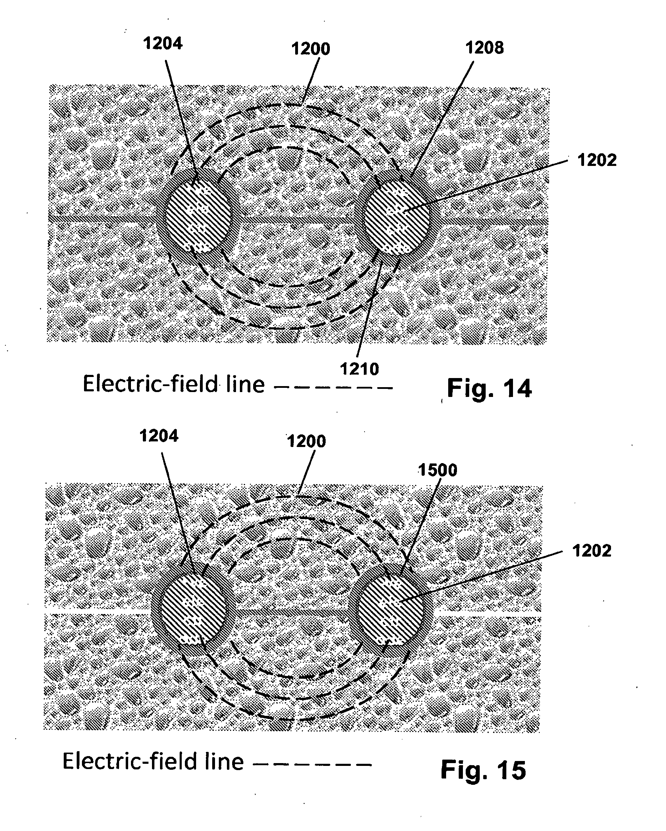

As such, compliant material, typically non-porous can be placed around the pins 1202, 1204. In the embodiment shown in Figure 14, the two pins 1202, 1204 are placed between two sheets of compliant material 1208, 1210. The sheets 1208, 1210 can then be placed between the porous material 1200 such that the gaps 1206 shown in Figure 13 become filled with the compliant material thereby helping to prevent the ingress of water into those gaps.

Figure 15 shows a slight modification to Figure 14 in which the pins 1202, 1204 are placed within a sleeve of compliant material 1500 which is then placed between portions of the compliant material 1200. Again, the compliant material, which is typically non-porous, fills the gap 1206.

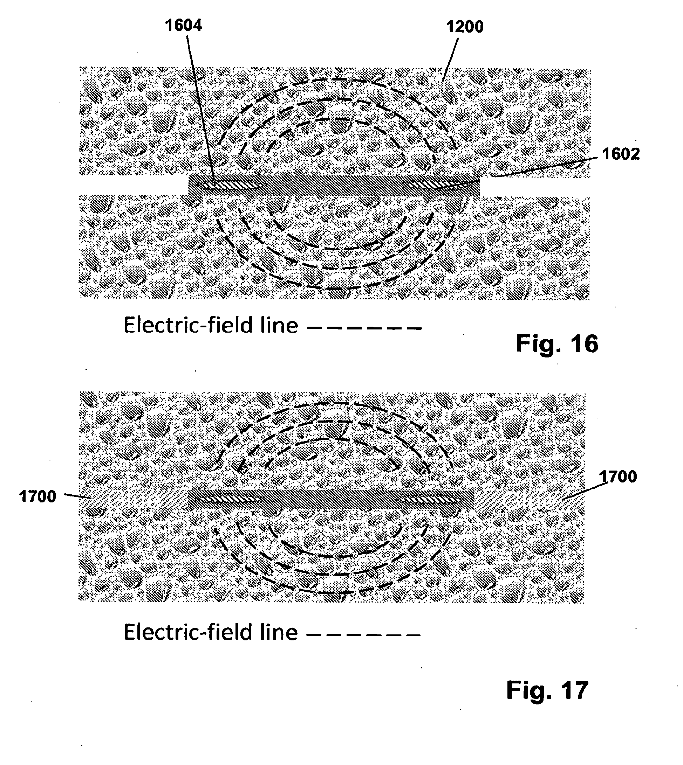

It the embodiments of Figures 14 and 15 it can be seen that the porous material portions 1200 have recesses therein to accommodate the electrodes 1202,1204. However, in the embodiment of Figure 16, flatter electrodes 1604, 1602 are used and thereby the porous material has no recess to accommodate the electrodes. In such an arrangement, the electrodes 1602, 1604 may be fabricated from a sheet material.

Figure 17 shows a further embodiment in which an adhesive 1700 is used to hold portions of the porous material in place. In such embodiments, it is believed advantageous to use the compliant, non-porous, material in the vicinity of the electrodes so that the porous material has a uniform property. Adhesives can penetrate into the porous material in an unknown, perhaps random, manner and alter the properties of the sensor.

## Claims

1. A matric potential sensor (6100) arranged to measure the matric potential of a medium and comprising a porous material portion (6102a, 6102b), arranged to absorb moisture from a medium into which, in use, it is inserted, and an electrode element (6104)
**characterised in that** a non-porous compliant material (606, 608) is provided between the porous material portion (6102a, 6102b) and the electrode element (6104).

2. A matric potential (6100) sensor according to claim 1 in which the non-porous compliant material (606, 608) is arranged in use to provide a layer between the porous material portion (6102a, 6102b), and the electrode element (6104) arranged to prevent the ingress of water.

3. A matric potential sensor (6100) according to claim 1 or claim 2 in which the porous material portion (6102a, 6102b) is a ceramic material.

4. A matric potential sensor (6100) according to any preceding claim in which the non-porous compliant material (606, 608) is closed cell foam or rubber which may typically be a silicone rubber.

5. A matric potential sensor (6100) according to any preceding claim in which the non-porous compliant material (606, 608) is provided as at least one sheet provided between the electrode element (6104) and the porous material portion (6102a, 6102b).

6. A matric potential sensor (6100) according to any of claims 1 to 4 in which the non-porous compliant material (606, 608) is provided as a sleeve around the electrode element (6104).

7. A matric potential sensor (6100) according to any preceding claim in which the non-porous compliant material (606, 608) is arranged, in use, to prevent water collecting adjacent to the electrode element (6104).

8. A matric potential sensor (6100) according to any preceding claim in which the porous material portion (6102a, 6102b) has pore sizes in the range of substantially 0.1µm to substantially 500µm and typically has a porosity in the range of substantially 20% to 50%.

9. A matric potential sensor (6100) according to any preceding claim in which there is provided two porous material portions (6102a and 6102b) and two portions of non-porous compliant material (606 and 608) in which the non-porous compliant material (606, 608) and/or porous material (6102a,6102b) are typically mirrored on each side of the electrode element (6104).

10. A matric potential sensor (6100) according to claim 9 in which one or more dimensions of the porous material (6102a, 6102b) in one of the two portions (6102a or 6102b) is varied when compared with the other portion of porous material (6102a or 6102b) and in particular the pore size in one of the portions (6102a or 6102b) may be varied in relation to the other of the portions.

11. A matric potential sensor (6100) according to any preceding claim in which the electrode element (6104) comprises a circuit board comprising one or more tracks (200, 202) wherein typically at least some of the tracks (200, 202) are arranged to form a plate of a capacitor.

12. A method of fabricating a matric potential sensor (6100) comprising placing a layer of non-porous compliant material (606, 608) between an electrode element (6104) and a portion of porous material (6102a, 6102b) arranged to absorb moisture from a medium into which, in use, it is inserted.

13. A moisture content meter including a matric potential sensor (6100) according to any of claims 1 to 11.

## Patentansprüche

1. Ein Matrixpotenzialsensor (6100), der so angeordnet ist, dass er das Matrixpotenzial eines Mediums messen kann, bestehend aus einem porösen Material-Teil (6102a, 6102b), der so angeordnet ist, dass er die Feuchtigkeit aus einem Medium absorbieren kann, in das er, bei Verwendung, eingesetzt ist, und ein Elektrodenelement (6104)
**dadurch gekennzeichnet, dass** ein nichtporöses konformes Material (606, 608) zwischen dem porösen Material-Teil (6102a, 6102b) und dem Elektrodenelement (6104) eingesetzt ist.

2. Ein Matrixpotenzialsensor (6100) gemäß Anspruch 1, in dem das nichtporöse konforme Material (606, 608) bei Verwendung so angeordnet ist, dass eine Schicht zwischen dem porösen Material-Teil (6102a, 6102b) und dem Elektrodenelement (6104) vorhanden ist, um das Eindringen von Wasser zu verhindern.

3. Ein Matrixpotenzialsensor (6100) gemäß Anspruch 1 oder Anspruch 2, in dem der poröse Material-Teil (6102a, 6102b) ein Keramik-Material ist.

4. Ein Matrixpotenzialsensor (6100) gemäß eines der vorhergehenden Ansprüche, in dem das nichtporöse konforme Material (606, 608) geschlossener Zellschaum oder Gummi ist, der typischerweise ein Silikongummi sein kann.

5. Ein Matrixpotenzialsensor (6100) gemäß eines der vorhergehenden Ansprüche, in dem das nichtporöse konforme Material (606, 608) so angeordnet ist, dass eine Schicht zwischen dem Elektrodenelement (6104) und dem porösen Material-Teil (6102a, 6102b) aus mindestens einer Folie besteht.

6. Ein Matrixpotenzialsensor (6100) gemäß eines der Ansprüche 1 bis 4, in dem das nichtporöse konforme Material (606, 608) als eine Hülse um das Elektrodenelement (6104) angebracht ist.

7. Ein Matrixpotenzialsensor (6100) gemäß eines der vorhergehenden Ansprüche, in dem das nichtporöse konforme Material (606, 608) bei Verwendung so angeordnet ist, dass das Ansammeln von Wasser am Elektrodenelement (6104) verhindert wird.

8. Ein Matrixpotenzialsensor (6100) gemäß eines der vorhergehenden Ansprüche, in dem der poröse Material-Teil (6102a, 6102b) Porengrößen im Wesentlichen im Bereich von 0,1µm bis im Wesentlichen 500µm und typischerweise eine Porosität im Wesentlichen im Bereich von 20% bis 50% aufweist.

9. Ein Matrixpotenzialsensor (6100) gemäß eines der vorhergehenden Ansprüche, in dem zwei poröse Material-Teile (6102a und 6102b) und zwei Teile eines nichtporösen konformen Materials (606 und 608) enthalten sind, wobei das nichtporöse konforme Material (606, 608) und/oder das poröse Material (6102a,6102b) typischerweise auf jeder Seite des Elektrodenelements (6104) gespiegelt werden.

10. Ein Matrixpotenzialsensor (6100) gemäß Anspruch 9, bei dem eine oder mehrere Abmessungen des porösen Materials (6102a, 6102b) in einem der beiden Teile (6102a oder 6102b) varüeren, wenn sie mit dem anderen Teil des porösen Materials (6102a oder 6102b) verglichen werden, insbesondere kann die Porengröße in einem der Teile (6102a oder 6102b) im Verhältnis zum anderen Teil variiert werden.

11. Ein Matrixpotenzialsensor (6100) gemäß eines der vorhergehenden Ansprüche, in dem das Elektrodenelement (6104) eine Schaltplatte umfasst, die aus einem oder mehreren Spuren (200, 202) besteht, wobei typischerweise mindestens einige der Spuren (200, 202) so angeordnet sind, dass sie die Platte eines Kondensators bilden.

12. Ein Verfahren zur Herstellung eines Matrixpotenzialsensors (6100) bestehend aus der Aufbringung einer Schicht aus nichtporösem konformem Material (606, 608) zwischen einem Elektrodenelement (6104) und einem Teil eines porösen Materials (6102a, 6102b), das so angeordnet ist, dass es die Feuchtigkeit aus einem Medium, in das es bei Verwendung eingesetzt ist, absorbiert.

13. Ein Feuchtigkeitsmessgerät, das einen Matrixpotenzialsensor (6100) gemäß eines der Ansprüche 1 bis 11 einschließt.

## Revendications

1. Un capteur de potentiel capillaire (6100) agencé de façon à mesurer le potentiel capillaire d'un milieu et comprenant une partie de matériau poreux (6102a, 6102b) agencée de façon à absorber une humidité provenant d'un milieu dans lequel, en utilisation, il est inséré, et un élément électrode (6104),
**caractérisé en ce qu'**un matériau souple non poreux (606, 608) est placé entre la partie de matériau poreux (6102a, 6102b) et l'élément électrode (6104).

2. Un capteur de potentiel capillaire (6100) selon la Revendication 1 dans lequel le matériau souple non poreux (606, 608) est agencé en utilisation de façon à fournir une couche entre la partie de matériau poreux (6102a, 6102b) et l'élément électrode (6104) et agencé de façon à empêcher l'entrée d'eau.

3. Un capteur de potentiel capillaire (6100) selon la Revendication 1 ou 2 dans lequel la partie de matériau poreux (6102a, 6102b) est un matériau céramique.

4. Un capteur de potentiel capillaire (6100) selon l'une quelconque des Revendications précédentes dans lequel le matériau souple non poreux (606, 608) est une mousse à alvéoles fermées ou un caoutchouc ou qui peut typiquement être un caoutchouc silicone.

5. Un capteur de potentiel capillaire (6100) selon l'une quelconque des Revendications précédentes dans lequel le matériau souple non poreux (606, 608) est fourni sous la forme d'au moins une feuille placée entre l'élément électrode (6104) et la partie de matériau poreux (6102a, 6102b).

6. Un capteur de potentiel capillaire (6100) selon l'une quelconque des Revendications 1 à 4 dans lequel le matériau souple non poreux (606, 608) est fourni sous la forme d'un manchon autour de l'élément électrode (6104).

7. Un capteur de potentiel capillaire (6100) selon l'une quelconque des Revendications précédentes dans lequel le matériau souple non poreux (606, 608) est agencé, en utilisation, de façon à empêcher une accumulation d'eau adjacente à l'élément électrode (6104).

8. Un capteur de potentiel capillaire (6100) selon l'une quelconque des Revendications précédentes dans lequel la partie de matériau poreux (6102a, 6102b) possède des tailles de pore dans la plage de sensiblement 0,1 µm à sensiblement 500 µm et possède typiquement un porosité dans la plage de sensiblement 20% à 50%.

9. Un capteur de potentiel capillaire (6100) selon l'une quelconque des Revendications précédentes dans lequel il est fourni deux parties de matériau poreux (6102a et 6102b) et deux parties de matériau souple non poreux (606 et 608), le matériau souple non poreux (606, 608) et/ou le matériau poreux (6102a,6102b) étant typiquement réfléchis sur chaque côté de l'élément électrode (6104).

10. Un capteur de potentiel capillaire (6100) selon la Revendication 9 dans lequel une ou plusieurs dimensions du matériau poreux (6102a, 6102b) dans l'une des deux parties (6102a ou 6102b) sont variées lorsqu'elles sont comparées à l'autre partie de matériau poreux (6102a ou 6102b) et, plus particulièrement, la taille de pore dans l'une des parties (6102a ou 6102b) peut être variée en relation avec l'autre des parties.

11. Un capteur de potentiel capillaire (6100) selon l'une quelconque des Revendications précédentes dans lequel l'élément électrode (6104) comprend une carte à circuits imprimés comprenant une ou plusieurs pistes (200, 202) où typiquement au moins certaines des pistes (200, 202) sont agencées de façon à former une plaque d'un condensateur.

12. Un procédé de fabrication d'un capteur de potentiel capillaire (6100) comprenant le placement d'une couche de matériau souple non poreux (606, 608) entre un élément électrode (6104) et une partie de matériau poreux (6102a, 6102b) agencée de façon à absorber une humidité provenant d'un milieu dans lequel, en utilisation, il est inséré.

13. Un dispositif de mesure de la teneur en humidité comprenant un capteur de potentiel capillaire (6100) selon l'une quelconque des Revendications 1 à 11.
